# EUROPEAN PATENT SPECIFICATION

(11) **EP 1 513 813 B1**
(45) Date of publication and mention of the grant of the patent: **02.11.2005**
(21) Application number: 03733973.6
(22) Date of filing: 22.05.2003
(51) Int. Cl.: C07D 211/14, A61K 31/445, A61P 3/00

(54) **OPIOID RECEPTOR ANTAGONISTS**
OPIOIDREZEPTORANTAGONISTEN
ANTAGONISTES DE RECEPTEUR D'OPIOIDES

(30) Priority: 30.05.2002 US 384603 P
(43) Date of publication of application: 16.03.2005
(73) Proprietor: ELI LILLY AND COMPANY, Indianapolis, Indiana 46285 (US)
(72) Inventor: MITCH, Charles, Howard, Columbus, IN 47203 (US); QUIMBY, Steven, James, Noblesville, IN 46060 (US)
(74) Representative: Burnside, Ivan John
(86) International application number: PCT/US2003/014540
(87) International publication number: WO 2003/101963

(56) References cited:
- EP-A- 1 072 592
- US-A- 4 891 379
- C. H. MITCH: "3,4-Dimethyl-4-(3-hydroxyphenyl)piperidin es: opioid antagonists with potent anorectant activity" J. MED. CHEM., vol. 36, no. 20, 1993, pages 2842-50, XP002249614
- WENTLAND M P ET AL: "8-Carboxamidocyclazocine analogues - redefining the structure-activity relationships of 2,6-methano-3-benzazocines" BIOORGANIC & MEDICINAL CHEMISTRY LETTERS, OXFORD, GB, vol. 11, no. 5, 12 March 2001 (2001-03-12), pages 623-626, XP004230076 ISSN: 0960-894X cited in the application

## Description

The present invention is in the field of medicinal chemistry. The invention relates specifically to compounds useful as opioid antagonists, methods of treatment, methods of using, and pharmaceutical compositions thereof.

### Background:

Three types of opioid receptors, mu, kappa, and delta opioid receptors are generally reported. Recent evidence points to the interactions between receptor dimer combinations of mu, kappa and/or delta receptors (called heterodimers) as also contributing to opioid activity. Opiate receptors and their normal regulation or lack thereof, has been implicated in disease states including irritable bowel syndrome, nausea, vomiting, pruritic dermatoses, depression, smoking and alcohol addiction, sexual dysfunction, stroke and trauma in animals. Therefore it is not surprising that the ability to antagonistically bind opioid receptors has been shown to produce ameliorative, preventative and/or treatment effects in animals including humans afflicted with one or more of these disease states.

More recently, antagonists of the opioid receptors have been found to increase metabolic energy consumption, and reduction of weight in obese rats while maintaining muscle mass. These findings indicate that an effective opioid antagonist may be useful in preventing, treating and or ameliorating the effect of obesity. Considering the percentage of the population that is obese in Western societies and the indirect costs associated with treating the effects and symptoms of obesity and Related Diseases, the impact of these findings cannot be overstated.

Though many opioid antagonists have been disclosed, the search continues for alternative and/or improved or more effective antagonists having an overall benefit to the patient with little or no major side effects. U.S Patent No. 4,891,379 discloses phenylpiperidine opioid antagonists useful for the treatment of diabetes and obesity. Clinical development of a compound claimed in U.S. Patent No. 4,191,771 was discontinued due to poor bioavalibility characteristics. Bicyclic analogs of phenyl piperidine have been prepared and reported as opioid antagonists by Wentland, et al., Biorganic and Medicinal Chemistry Letters 11 (2001) 623-626; see also Wentland, et al., Biorganic and Medicinal Chemistry Letters 11 (2001) 1717-1721. Finally, European Patent application number 1 072592A2 filed May 18, 2000, discloses phenylpiperidine compounds of formula I 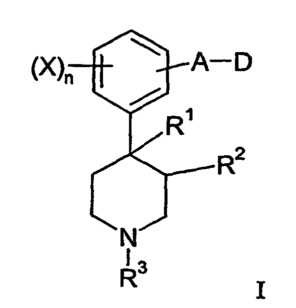 wherein A, D, R¹, R², R³ X, and n have meanings given in the description, which are useful in the prophylaxis and in the treatment of diseases mediated by opioid receptors such as pruritus.

Not withstanding these and other disclosures of compounds useful as opioid receptor antagonists, there remains an unmet medical need for safe, effective and/or alternate treatment or prophylaxis of diseases associated with opioid receptors, particularly obesity and Related Diseases.

### SUMMARY OF THE INVENTION

The present invention relates to a compound of the formula (I) 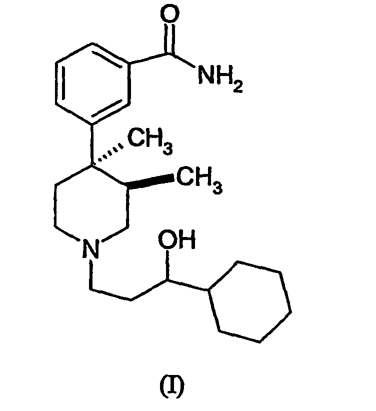 or a pharmaceutically acceptable salt, solvate, enantiomer, racemate, diastereomers or mixtures thereof.

The present invention also provides a method of using a compound of formula I for the prevention, treatment and/or amelioration of the symptoms of obesity and Related Diseases.

The present invention also provides a pharmaceutical formulation comprising a compound of formula I in association with a carrier, diluent and/or excipient

The present invention provides a compound of formula I having improved efficacy and bio-availability compared to compounds disclosed in U.S Patent 4,891,379 and European Patent application EP 1,072,592 A2.

The present invention relates to the use of a compound of formula I for the preparation of a medicament for the treatment and/or prophylaxis of obesity and Related Diseases including eating disorders (bulima, anorexia nervosa, etc.), diabetes, diabetic complications, diabetic retinopathy, sexual/reproductive disorders, depression, anxiety, epileptic seizure, hypertension, cerebral hemorrhage, conjestive heart failure, sleeping disorders, atherosclerosis, rheumatoid arthritis, stroke, hyperlipidemia, hypertriglycemia, hyperglycemia, and hyperlipoproteinenamia, substance abuse, drug overdose, compulsive behavior disorders (such as paw licking in dog), addictive behaviors such as gambling.

The present invention provides a compound of formula (I) useful for the manufacture of a medicament for the treatment, prevention and/or amelioration of symptoms associated with obesity, Related Diseases.

The present invention provides a compound of formula I useful in the treatment of obesity and related diseases with reduced potential for inhibition of cytochrome P450 enzyme.

In another embodiment, the present invention provides a compound of formula I or a pharmaceutically acceptable salt, solvate, enantiomer, racemate, diastereomers or mixtures thereof, useful as an appetite suppressant

### DETAILED DESCRIPTION OF THE INVENTION

The term "suitable solvent" refers to any solvent, or mixture of solvents, inert to the ongoing reaction that sufficiently solubilizes the reactants to afford a medium within which to effect the desired reaction.

The term "mutual solvent" means a solvent that is used to dissolve two or more components of a reaction or mixture separately prior to reaction or mixing, that is a solvent common to more than one reagents or components of a mixture.

As used herein, the term "patient" includes human and non-human animals such as companion animals (dogs and cats and the like) and livestock animals. Livestock animals are animals raised for food production. Ruminants or "cud-chewing" animals such as cows, bulls, heifers, steers, sheep, buffalo, bison, goats and antelopes are examples of livestock. Other examples of livestock include pigs and avians (poultry) such as chickens, ducks, turkeys and geese. Also included are exotic animals used in food production such as alligators, water buffalo and ratites (e.g., emu, rheas or ostriches).

The preferred patient of treatment or prevention is a human.

The terms "treating" and "treat", as used herein, include their generally accepted meanings, *i*.*e*., preventing, prohibiting, restraining, alleviating, ameliorating, slowing, stopping, or reversing the progression or severity of a pathological condition, or sequela thereof, described herein.

The terms "preventing", "prevention of", "prophylaxis", "prophylactic" and "prevent" are used herein interchangeably and refer to reducing the likelihood that the recipient of a compound of formula I will incur or develop any of the pathological conditions, or sequela thereof, described herein.

As used herein, the term "effective amount" means an amount of a compound of formula I that is sufficient for treating a condition, or detrimental effects thereof, herein described, or an amount of a compound of formula I that is sufficient for antagonizing the opioid receptors to achieve the objectives of the invention.

The term "pharmaceutically acceptable" is used herein as an adjective and means substantially non-deleterious to the recipient patient.

The term "formulation", as in pharmaceutical formulation, or "pharmaceutical composition" is intended to encompass a product comprising the active ingredient (compound of formula I), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical formulations of the present invention encompass any composition made by admixing a compound of the present invention and a pharmaceutical carrier, or a compound of the formula I and a pharmaceutically acceptable co-antagonist of opioid receptors useful for the treatment and/or prevention of obesity or Related Diseases where antagonism of opioid receptors may be beneficial.

The terms "obesity and Related Diseases" or "Related Diseases" as used herein refers to such symptoms, diseases or conditions caused by, exacerbated by, induced by or adjunct to the condition of being obese. Such diseases, conditions and/or symptoms include but are not limited to eating disorders (bulima, anorexia nervosa, etc.), diabetes, diabetic complications, diabetic retinopathy, sexual/reproductive disorders, depression, anxiety, epileptic seizure, hypertension, cerebral hemorrhage, congestive heart failure, sleeping disorders, atherosclerosis, rheumatoid arthritis, stroke, hyperlipidemia, hypertriglycemia, hyperglycemia, and hyperlipoproteinenamia.

The term "cytochrome P450 enzyme" as used herein refers to the family of enzymes comprised of the cytochrome P450 system often called cyotchrome P's. It has become increasingly clear that inhibition of an enzyme or enzymes from this family is associated with deleterious effects which could be life threatening. For example, inhibition of the enzyme Cyp2D6, a member of the cytochrome P450 family, may cause serious drug-drug interactions and/or overdoses particularly in cases where patients are taking multiple medications. Thus, the present invention also relates to the use of a compound of formula I for the treatment or prevention of obesity and Related Diseases while minimizing undesirable drug-drug interactions in a patient who is also under medication with other drug(s) comprising administering a therapeutically effective amount of a compound of formula I to said patient.

The compound of the invention as illustrated in formula I occurs as the trans stereochemical isomer by virtue of the substituents at the 3- and 4-positions. More specifically, the group CH₃, at the 3-position is situated in a trans configuration relative to the CH₃ group at the 4-position. As such, the compound can exist as the trans (+) isomer of the formula 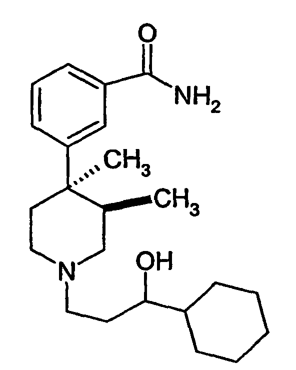 or the trans (-) isomer of the formula

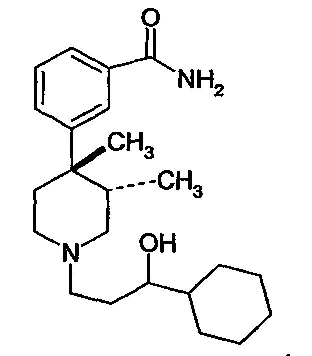

The present invention comtemplates the individual trans (+) and (-) stereoisomers, as well as the mixture of the trans stereoisomers.

Also, for the group -CH₂CH₂C(OH)cyclohexyl there is the possibility of a chiral center, i.e. the carbon atom attached to the OH group is asymmetric. Therefore, the compound can further exist as the individual R or S stereoisomers, or the mixture of the isomers, and all are contemplated within the scope of the compounds of the present invention. A most preferred compound of the invention is trans(+)-1-[-3S-(3-hydroxy-3-cyclohexylpropyl)]-3(R),4(R)-dimethyl-4-(3-phenylcarboxamido) piperidine.

The compound of formula I forms pharmaceutically acceptable acid addition salts with a wide variety of inorganic and organic acids. The compound of formula I preferably exists as a pharmaceutically acceptable salt. More preferred is the hydrochloride, or the bisulfate salt of the compound of formula I.

In another embodiment, the compound(s) of the present invention has shown antiorexigenic effects, and is thus useful as an appetite suppressant. Reduction of food intake over a period of time has been observed with rats fed a diet containing a compound of the invention. Interestingly the reduction in food intake was found to be more significant at each point in time for the compound of the present invention than with the clinical trial compound disclosed in U.S. Patent number 4,891,379.

### Preparing the Compound of the Invention

The compound of the present invention may be prepared by a variety of procedures known to one of skill in the art. The preferred procedure involves transforming the OH group at the 3-position of the phenyl group of 3-,4-dimethyl-4-(3-hydroxyphenyl)piperidine into a good leaving group for example, by triflate formation or mesylate formation followed by a subsequent nucleophilic attack by a carbonyl group or synthon thereof. The carbonyl group or synthon thereof is then converted to the amide compound of formula I. The starting material 3,4-dimethyl-4-(3-substituted phenyl)piperidine (1) is reacted with an appropriate acylating agent (2) to provide the corresponding intermediate (3) which is reduced to the intermediate (4) and then converted to the compound of the present invention (6) via a triflate intermediate (5) under standard conditions as shown in Scheme 1.

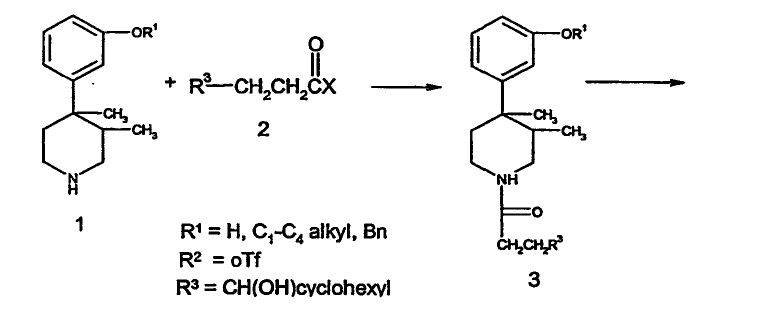

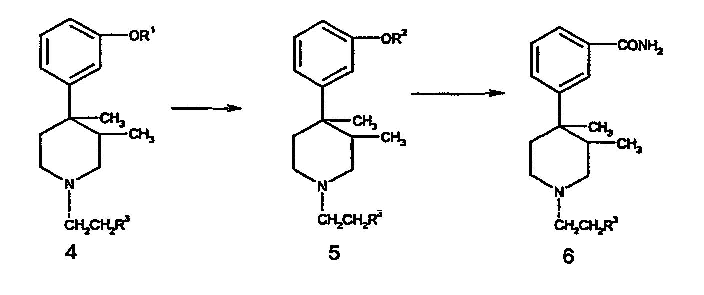

The first step of the above-described process wherein X is hydroxy, necessitates the use of coupling reagents commonly employed in the synthesis of peptides. Examples of such coupling reagents include the carbodiimides such as N,N'-dicyclohexylcarbodiimide, N,N'-diisopropylcarbodiimide, or N,N'-diethylcarbodiimide; the imidazoles such as carbonyldiimidazole; as well as reagents such as N-ethoxycarbonyl-2-ethoxy-1,2-dihydroquinoline (EEDQ). The direct coupling of a substituted carboxylic acid and a 3-substituted-4-methyl-4-(3-substitutedphenyl)piperidine (1) is carried out by adding about an equimolar quantity of the piperidine starting material to a solution of the carboxylic acid in the presence of an equimolar quantity or slight excess of coupling reagent. The reaction generally is carried out in an unreactive organic solvent such as dichloromethane or N,N-dimethylformamide, and usually is complete within about twenty-four hours when conducted at a temperature of about 0 °C to about 30 °C. The product is then typically isolated by filtration. The acylated product (3) thus formed may be further purified, if needed, by any of several routine methods, including crystallization from common solvents, chromatography over solid supports such as silica or alumina, and related purification techniques.

The reaction (as in Scheme 1) wherein X is other than hydroxy is conducted as follows. The preferred leaving group in this reaction is where X is halogen, especially chloro. The reaction can be carried out by combining the substituted carboxylic acid derivative with about an equimolar quantity of the 3-substituted-4-methyl-4-(3-substituted phenyl)piperidine in a solvent such tetrahydrofuran, diethyl ether, dichloromethane, dioxane, dimethylsulfoxide, N,N-dimethylformamide, benzene, toluene, and the like. If desired, a base can be utilized in the acylation reaction when X is halogen to act as an acid scavenger. Commonly used bases include sodium carbonate, potassium carbonate, pyridine, triethylamine and related bases. Bases such as pyridine act as their own solvent and need no additional solvent. The reaction generally is substantially complete after about two to about 200 hours when carried out at a temperature of about 20 °C. to about 200 °C, preferably from about 30 °C to about 100 °C The product of the reaction may be isolated by simply removing the reaction solvent, for instance by evaporation under reduced pressure. Also, the reaction mixture may be added to water, and the product collected by filtration or extracted into a water immiscible solvent. The compound (3) thus isolated can be further purified, if desired, by any of several well-known techniques. A suitable R¹ group for the above reaction is methyl, ethyl or the like.

The acylated intermediate (3) prepared as above is reduced according to standard procedures to provide the intermediate (4) useful in preparing the present compounds. Typical reducing agents suitable for use include the hydride reducing agents such as lithium aluminum hydride and sodium bis(2-methoxyethoxy)aluminum hydride, which is preferred. Typically, an excess of reducing agent is combined with the acylated intermediate in a mutual solvent. The reaction is substantially complete after about one to about 12 hours when conducted at a temperature in the range of about 20°C to about 100 °C. The desired intermediate (4) may then be isolated by procedures well known to those of ordinary skill in the art.

The intermediate (4) may also be prepared by the direct substitution of a halogen substituted compound with the 3,4-dimethyl-4-(3-substituted phenyl)piperidine intermediate. This reaction is represented by the following scheme (2) wherein R¹ is hydrogen, C₁-C₄ alkyl, or benzyl; R³ is cyclohexyl; Z is -CH(OH)-; and Y is halogen. 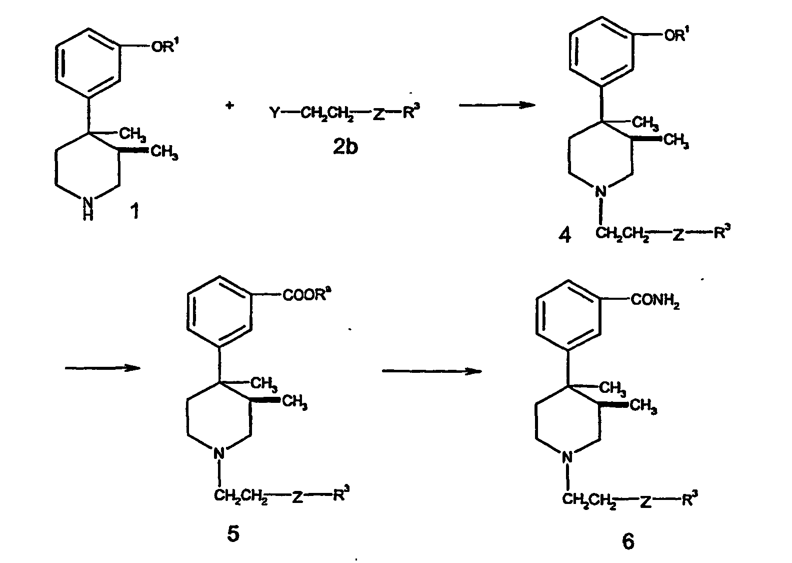 The above reaction (scheme 2) is conducted by combining approximately equimolar amounts of the two starting materials (compounds 1 and 2b) (salts, stereoisomers, and racemates thereof) in a mutual solvent. A slight excess of the halogen-substituted compound (2b) may be employed to ensure complete reaction. Typical mutual solvents suitable for use in this reaction include aprotic solvents such as N,N-dimethylformamide and the like. Further, the reaction is preferably conducted in the presence of a base, such as sodium bicarbonate, which acts as an acid scavenger for the hydrohalic acid, which is formed as a by-product of the reaction. The reaction is generally complete after about 30 minutes to 24 hours when conducted at a temperature in the range of about 40 °C to about 100 °C. The product is isolated and purified, if needed, by standard procedures or isolation procedures described herein.

Other methods of preparing the intermediates (3) and/or (4) or analogs thereof, are disclosed in U.S Patent Nos. 4,081,450, and 4,191,771, European Patent application EP.1 072 592 A2 and references disclosed therein.

The intermediate (4) however prepared, is activated at the hydroxy group by reaction with a methane sulfonic anhydride, triflic anhydride, or other reagents known to one of skill in the art to convert the hydroxyl group to a good leaving group forming an isolatable intermediate triflate or mesylate. The intermediate triflate for example, is converted to the compound (5) by nucleophilic attack of a carbonyl group or synthon thereof followed by esterification. In a preferred mode of reaction, the carbonyl group is inserted by use of palladium reagents (carbonyl insertion reaction) often accompanied by *in-situ* esterification to afford the ester (5)(wherein R^{a} is C₁-C₄ alkyl or benzyl). The ester (5) is converted to the amide (6) by sealed tube ammonolysis conditions or other reaction conditions known to one of skill in the art. An alternate route involving direct conversion of a triflate intermediate to a carboxamide is illustrated in Wentland, et al., Biorganic and Medicinal Chemistry Letters 11 (2001) 623-626 and also in Wentland, et al., Biorganic and Medicinal Chemistry Letters 11 (2001) 1717-1721. For the compound of the present invention, the route involving converting the triflate to an isolable ester intermediate was found to be most workable and therefore preferred. Additional information for preparing the compound of formula I is available in the experimental section.

Salts of piperidines are prepared by methods commonly employed for the preparation of amine salts. In particular, acid addition salts of the piperidines are prepared by reaction of the piperidine with an appropriate acid ofpKa less than about 4, generally in an unreactive organic solvent. Suitable acids include mineral acids such as hydrochloric, hydrobromic, hydriodic, sulfuric, phosphoric, and like acids. Organic acids are also used, for example acetic acid, p-toluenesulfonic acid, chloroacetic acid, and the like. The usual solvents used in the reaction include acetone, tetrahydrofuran, diethyl ether, ethyl acetate, and the like. Quaternary salts can be prepared in generally the same way by reaction of the piperidne with an alkylsulfate or alkyl halide, for example, methyl sulfate, methyl iodide, ethyl bromide, propyl iodide, and the like.

The 3,4-dimethyl-4-(3-hydroxy- or -alkanoyloxyphenyl)piperidine derivative (1) employed as starting material in the synthesis of the compound of the present invention is prepared by the general procedures taught by Zimmerman in U.S. Pat. Nos.4,081,450, and 4,191,771 and references therein, and procedures disclosed in European Patent Application No. 1 072 592 A2 and known and applicable modifications thereof. The above references for the preparation of the starting material 3,4-dimethyl-4-(3-hydroxy- or -alkanoyloxyphenyl)piperidine derivative (1), are incorporated by reference in their entirety where applicable.

As noted above, the compounds of the present invention may exist as the resolved stereoisomers. The preferred procedure employed to prepare the resolved starting materials used in the synthesis of these compounds includes treating a 1,3-dialkyl-4-methyl-4-(3-alkoxyphenyl)piperidine with either (+)- or (-)-dibenzoyl tartaric acid to provide the resolved intermediate. This compound is dealkylated at the 1-position with vinyl chloroformate and finally converted to the desired 4-(3-hydroxyphenyl)piperidine isomer.

As will be understood by one skilled in the art, the individual trans stereoisomers of the compound of the present invention may also be isolated with either (+)- or (-)-dibenzoyl tartaric, or other resolving agents and/or techniques known to one of skill in the art, from the corresponding racemic mixture of the compound of the invention.

### EXPERIMENTAL

The following Example illustrates a method for the preparation of the compound of the present invention.

### Example

### Synthesis of 3-[1-(3-Cyclohexyl-3-hydroxy-propyl)-3,4-dimethyl-piperidin-4-yl]-benzamide

### Synthesis of Trifluoro-methanesulfonic acid 3-[1-(3-cyclohexyl-3-hydroxy-propyl)-3,4-dimethyl-piperidin-4-yl]-phenyl ester

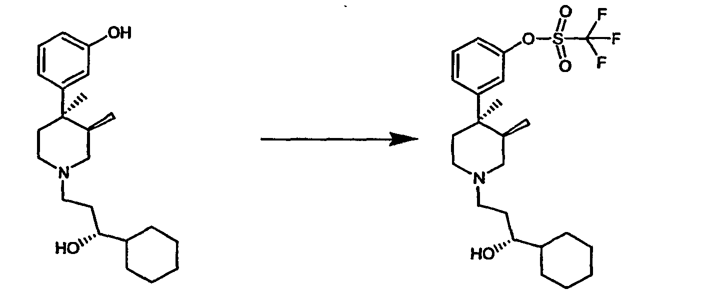

A 250mL round bottom flask equipped with an addition funnel and nitrogen inlet was charged with 2g (5.8mmol) of trans-3,4-dimethyl-4-(3-hydroxyphenyl) piperidine prepared following the procedure disclosed in U.S. Patent number 4,191,771. The flask was then charged with 3.2mL (23.0mmol) of triethylamine, and 35mL of dichloromethane. While stirring at room temperature, 2.3g (6.4mmol) of N-phenyltrifluoromethanesulfonimide in 5mL of dichloromethane was added to the reaction dropwise via an addition funnel. The reaction mixture was stirred at room temperature for four hours. The reaction mixture was concentrated on a rotary evaporator to yield 4.3g of crude product. The crude product was purified by flash chromatography on silica gel eluting with 1% conc. ammonium hydroxide / 10% ethanol in chloroform to yield 2.0g (4.2mmol) of trifluoro-methanesulfonic acid 3-[1-(3-cyclohexyl-3-hydroxy-propyl)-3,4-dimethyl-piperidin-4-yl]-phenyl ester. Electrospray MS M+1 ion = 478.6, H¹ NMR.

### Synthesis of 3-[1-(3-Cyclohexyl-3-hydroxy-propyl)-3,4-dimethyl-piperidin-4-yl]-benzoic acid methyl ester

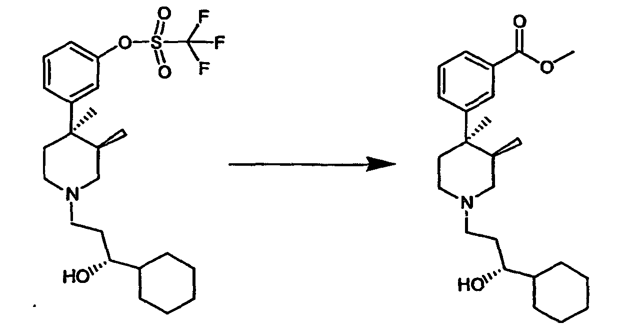

A 100mL sealed tube was charged with 2g (4.2 mmol) of trifluoro-methanesulfonic acid 3-[1-(3-cyclohexyl-3-hydroxy-propyl)-3,4-dimethyl-piperidin-4-yl]-phenyl ester, 94mg (0.42mmol) of palladium acetate, 465mg (0.84mmol) of dppf, 1.29mL (9.2mmol) of triethylamine, 20mL of methanol, and 32mL of dimethylsulfoxide (DMSO). Carbon monoxide was bubbled subsurface into the reaction for about ten minutes. The tube was sealed and heated at 65°C for four hours. The reaction mixture was concentrated on a rotary evaporator to a residue. Water (approximately 100mL) was added to the residue followed by extraction of the organic phase with ethyl acetate (3x100mL). The organic extracts were dried over sodium chloride/sodium sulfate, filtered, and then concentrated to yield 2.4g of crude product. The crude product was purified by flash chromatography on silica gel eluting with 1% conc. ammonium hydroxide / 10% ethanol in chloroform to yield 0.7g (1.8mmol) of 3-[1-(3-cyclohexyl-3-hydroxy-propyl)-3,4-dimethyl-piperidin-4-yl]-benzoic acid methyl ester. HPLC-MS = 100% M+1 ion 388.23.

### Synthesis of 3-[1-(3-Cyclohexyl-3-hydroxy-propyl)-3,4-dimethyl-piperidin-4-yl]-benzamide

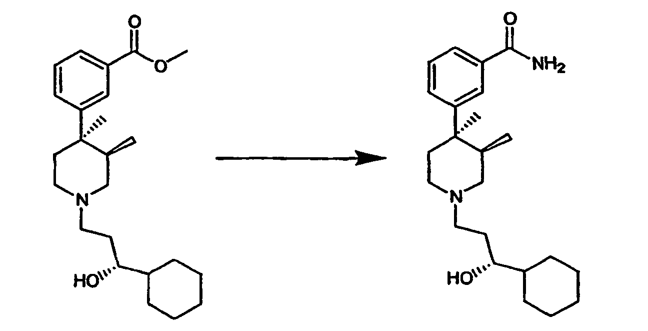

A reaction tube was charged with 0.7g (1.8mmol) of 3-[1-(3-cyclohexyl-3-hydroxy-propyl)-3,4-dimethyl-piperidin-4-yl]-benzoic acid methyl ester, 5mg of sodium cyanide, and 30mL of methanol. Ammonia was bubbled subsurface into the reaction for ten minutes then the tube was sealed at heated at 85°C for sixteen hours. The reaction was driven to completion or substantial completion by daily addition of sodium cyanide and ammonia over a period of seven days or until the reaction was satisfactorily complete by HPLC analysis. Care is taken to cool the tube to between 0 °C and room temperature before addition of each new batch of sodium cyanide and ammonia. The reaction mixture is concentrated on a rotary evaporator to yield 0.6g of crude product. The crude product is purified by flash column chromatography on silica gel eluting with 1% conc. ammonium hydroxide / 10% ethanol in chloroform to yield 260mg (0.7mmol) of 3-[1-(3-Cyclohexyl-3-hydroxy-propyl)-3,4-dimethyl-piperidin-4-yl]-benzamide. HPLC = 98%, Electrospray MS M+1 ion = 373.1, H¹ NMR.

### Method of Using the Invention

As noted above, the compound of the present invention is useful in blocking the effect of agonists at mu, kappa, and/or delta receptors. As such, the present invention also provides a method for blocking a mu, kappa, delta or receptor combination (heterodimer) thereof in mammals comprising administering to a mammal requiring blocking of a mu, kappa, delta or combinations of mu, kappa, and/or delta receptors, a receptor blocking dose of a compound of formula I.

The term "receptor blocking dose", as used herein, means an amount of compound necessary to block a mu, kappa, or delta or receptor combination (heterodimer) thereof receptor following administration to a mammal requiring blocking of a mu, kappa, or delta or receptor combination (heterodimer) thereof receptor. The active compounds are effective over a wide dosage range. For example, dosages per day will normally fall within the range of about 0.05 to about 250 mg/kg of body weight. In the treatment of adult humans, the range of about 0.5 to about 100 mg/kg, in single or divided doses, is preferred. However, it will be understood that the amount of the compound actually administered will be determined by a physician in light of the relevant circumstances, including the condition to be treated, the choice of compound to be administered, the age, weight, and response of the individual patient, the severity of the patient's symptoms, and the chosen route of administration, and therefore the above dosage ranges are not intended to limit the scope of the invention in any way. The compounds may be administered by a variety of routes such as the oral, transdermal, subcutaneous, intranasal, intramuscular and intravenous routes.

A variety of physiologic functions have been shown to be subject to influence by mu, kappa, or delta or receptor combination (heterodimers) thereof in the brain. As such, the compound of the present invention is believed to have the ability to treat a variety of disorders in mammals associated with these receptors such as eating disorders, opioid overdose, depression, smoking, alcoholism, sexual dysfunction, shock, stroke, spinal damage and head trauma. As such, the present invention also provides methods of treating the above disorders at rates set forth above for blocking the effect of agonists at a mu, kappa, delta or receptor combination (heterodimer) thereof. The compound of the present invention has been found to display excellent activity in an opioid receptor binding assay which measures the ability of the compounds to block the mu, kappa, delta or receptor combination (heterodimer) thereof.

Futhermore, the compound of the present invention has been found to exhibit an unexpected and significant increase in efficacy compared to compounds disclosed in published European Patent application number 1 072592A2 (i.e. compound of example 2). The compound of the present invention is also unique because in addition to increased or comparable efficacy over disclosed compounds, it also provides significantly improved bioavailability characteristics. The increased efficacy and the superior bioavailability characteristics of the present compound were neither appreciated nor suggested by the prior art. Thus the compound of the present invention is believed to provide truly superior and unexpected advantages over the prior art (see Table (1) *infra*).

The assay for biological activity i.e. binding affinity was conducted using the following procedure.

### GTPγS Binding Assay

An SPA - based GTPgS assay format was developed based on previous opioid (Emmerson et al., J. Pharm Exp Ther 278,1121,1996; Horng et al., Society for Neuroscience Abstracts, 434.6, 2000) and muscarinic (DeLapp et al., JPET 289, 946, 1999) assay formats. Membranes were resuspended in 20 mM HEPES, 100 mM NaCl, 5 mM MgCl2, 1 mM DTT, and 1 mM EDTA. Fifty mL of GTPγ[35S], compound, membrane suspension (20 microgram/well), and wheat germ agglutinin coated SPA beads (1mg/well) were added to clear bottom 96 well assay plates. GDP (200 mM) was added to the membrane solution prior to addition to the assay plates. Plates were sealed and incubated for four hours at room temperature then placed in a refrigerator overnight to allow the beads to settle. Signal stability at 4 °C was determined to be > 60 hours. Plates were warmed to room temperature and counted in a Wallac Microbeta scintillation counter. For antagonist assays, specific agonists were added at the following concentrations: (MOR) DAMGO 1 micromolar, (DOR) DPDPE 30 nM, (KOR) U69593 300 nM. Kb's were determined by Cheng-Prusoff equation (Cheng and Prusoff, 22,3099 1973).

Table 1 provides a summary of in vitro activity in a GTP-γ-S functional antagonist assay. This data shows that the compound of formula I is at least 2 fold more potent than the compound of formula (II) (the closest compound exemplified in European Patent application No. 1072592 A2), and comparable potency compared to compound (III), a previous clinical trial candidate discontinued for unacceptable bioavailability which is disclosed and claimed in U.S. Patent No. 4,891,379.

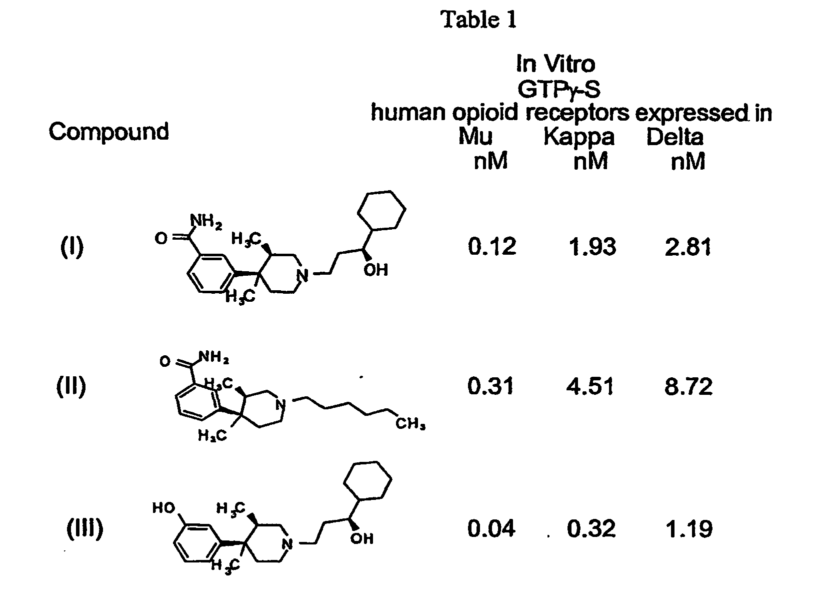

Table 2 provides data showing that the compound of formula (I) also shows better bioavailability than the compound of formula (III), a previous clinical trial candidate disclosed and claimed in U.S. Patent No. 4,891,379.

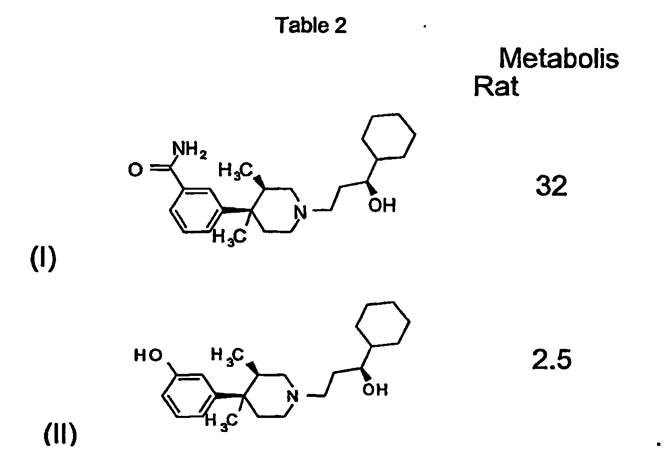

The compound of formula I, in addition to increased efficacy and bio-availability compared to previously disclosed compounds, also exhibits a significantly reduced potential for inhibiting the cytochrome P450 enzyme system, a surprising and unexpected finding that spells improved safety and reduced potential for drug-drug interactions etc. The reduced potential for inhibition of cytochrome P450 was discovered using a standard assay that monitors the compound's ability to inhibit Cyp2D6 a member of the cytochrome P450 family of enzymes. The protocol and comparative results are provided below.

### Assay for Inhibition of CYP2D6 activity

The inhibition of human Cytochrome 2D6 (CYP2D6) activity was studied using a validated high through-put screening assay. A 3 uL aliquot of an 8 mM stock solution of compound was delivered to 397 uL of pH 7.4 (50mM) phosphate buffer resulting in an initial concentration of 60uM. The compound dose solutions were prepared by serial dilutions to produce concentrations of 60, 19.4, 6.28, 2.03, 0.66, 0.21, 0.069, and 1 uM. A 6 mM NaDPH stock solution was prepared by addition of 100mg of NaDPH to 20 mL of pH 7.4 buffer. A 600uL aliquot of Human liver Microsomes (HLM - 20.0 mg/ml) was added to 20 ml of pH 7.4 phosphate buffer to produce a 0.6 mg/ml solution of HLM. To the HLM mixture was added 120 uL of a 10 mM bufurolol solution (CYP2D6 substrate) producing a final bufuralol concentration of 60 uM. To each assay plate was added 100 uL of the compound dose solutions, 25 uL NaDPH and 25 uL HLM/bufurolol solution. Samples were incubated at 37°C for 10 minutes and the reaction was quenched with 25 uL of a 2% perchloric acid solution, followed by centrifugation at 3200 rpm for 30 min. The resulting supernatant was assayed for bufurolol concentrations using a Turbo Ion Spray API 150EX MS method. The calculated IC50 value represents the compound concentration that results in a 50% reduction in burfurolol consumption. Table 3 below provides a comparative data for inhibition of CyP2D6

**Table 3**

| Compound # | Cyp2D6 IC₅₀ |
|---|---|
| 1 | 38.96 |
| II | 2.46 |
| III | 9.27 |

Table 3 shows that the compound of formula I is nearly 16 times less likely to cause inhibition of the cytochrome P450 enzyme compared to the prior clinical trial candidate compound (II) claimed in U.S. Patent No. 4,891,379. Furthermore, the data shows that the compound of formula (I) is over 4 times less likely to inhibit the cytochrome P450 enzyme system compared to the compound of formula (III) which is disclosed as example 2 in European Patent application number 1072592 A2 published January 31, 2001.

### Antiorexigenic effect

Compounds were tested for effects on food consumption in male Long-Evans rats which had been fasted for 18 hours prior to testing. The weight of food consumed was measured for groups of 6 rats treated with test substance and compared to the food consumed by an untreated control group of 6 animals. Oral administration of a 3 mg/kg dose of a compound of formula I resulted in a statistically significant inhibition of cumulative food consumed, as measured over 1hour, 2 hour and 4 hour time periods. Oral administration of a 3 mg/kg dose of the previous clinical trial compound (II) disclosed in U.S Patent No. 4,891,379 did not produce statistically significant inhibition of food consumption over the same time periods.

### Formulation

While it is possible to administer a compound of the invention directly without any formulation, the compounds are preferably employed in the form of a pharmaceutical formulation comprising a pharmaceutically acceptable carrier, diluent or excipient and a compound of the invention. Such compositions will contain from about 0.1 percent by weight to about 90.0 percent by weight of a present compound. As such, the present invention also provides pharmaceutical formulations comprising a compound of the invention and a pharmaceutically acceptable carrier, diluent or excipient therefor.

In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material that acts as a vehicle, excipient or medium for the active ingredient. Thus, the composition can be in the form of tablets, pills, powders, lozenges, sachets, cachets, elixirs, emulsions, solutions, syrups, suspensions, aerosols (as a solid or in a liquid medium, and soft and hard gelatin capsules.

Examples of suitable carriers, excipients, and diluents include lactose, dextrose, sucrose, sorbitol, mannitol, starches, gum acacia, calcium phosphate, alginates, calcium silicate, microcrystalline cellulose, polyvinylpyrrolidone, cellulose, tragacanth, gelatin, syrup, methyl cellulose, methyl- and propylhydroxybenzoates, talc, magnesium stearate, water, and mineral oil. The formulations may also include wetting agents, emulsifying and suspending agents, preserving agents, sweetening agents or flavoring agents. The formulations of the invention may be formulated so as to provide quick, sustained, or delayed release of the active ingredient after administration to the patient by employing procedures well known in the art.

For oral administration, a compound of this invention ideally can be admixed with carriers and diluents and molded into tablets or enclosed in gelatin capsules.

The compositions are preferably formulated in a unit dosage form, each dosage containing from about 1 to about 500 mg, more usually about 5 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

In order to more fully illustrate the operation of this invention, the following formulation examples are provided. The examples are illustrative only, and are not intended to limit the scope of the invention. The formulations may employ as active compounds any of the compounds of the present invention.

### FORMULATION 1

Hard gelatin capsules are prepared using the following ingredients:

| Compound | Amount per capsule (mg) | Concentration by weight (%) |
|---|---|---|
| Cyclohexyl-3-hydroxy-propyl)-3,4-dimethyl-piperidin-4-yl]-benzamide | 250 | 55 |
| Starch dried | 200 | 43 |
| Magnesium stearate | 10 | 2 |

The above ingredients are mixed and filled into hard gelatin capsules in 460 mg quantities.

### FORMULATION 2

Capsules each containing 20 mg of medicament are made as follows:

| Compound | Amount per capsule (mg) | Concentration by weight (%) |
|---|---|---|
| Cyclohexyl-3-hydroxy-propyl)-3,4-dimethyl-piperidin-4-yl]-benzamide | 20 | 10 |
| Starch | 89 | 44.5 |
| Microcrystalline cellulose | 89 | 44.5 |
| Magnesium stearate | 2 | 1 |

The active ingredient, cellulose, starch and magnesium stearate are blended, passed through a No. 45 mesh U.S. sieve and filled into a hard gelatin capsule.

### FORMULATION 3

Capsules each containing 100 mg of active ingredient are made as follows:

| Compound | Amount per capsule (mg) | Concentration by weight (%) |
|---|---|---|
| Cyclohexyl-3-hydroxy-propyl)-3,4-dimethyl-piperidin-4-yl]-benzamide | 100 | 30 |
| Polyoxyethylene Sorbitan monooleate | 50mcg | 0.02 |
| Starch powder | 250 | 69.98 |

The above ingredients are thoroughly mixed and placed in an empty gelatin capsule.

### FORMULATION 4

Tablets each containing 10 mg of active ingredient are prepared as follows:

| Compound | Amount per capsule (mg) | Concentration by weight (%) |
|---|---|---|
| Cyclohexyl-3-hydroxy-propyl)-3,4-dimethyl-piperidin-4-yl]-benzamide | 10 | 10 |
| Starch | 45 | 45 |
| Microcrystalline cellulose | 35 | 35 |
| Polyvinylpyrrolidone (as 10% solution in water) | 4 | 4 |
| Sodium carboxymethyl starch | 4.5 | 4.5 |
| Magnesium stearate | 0.5 | 0.5 |
| talc | 1 | 1 |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granule so produced is dried at 50-60 °C and passed through a No. 18 mesh U.S. sieve. The sodium carboxymethyl starch, magnesium stearate and talc, previously passed through a No. 60 mesh U.S. sieve, are then added to the granule which, after mixing, is compressed on a tablet machine to yield a tablet weighing 100 mg.

### FORMULATION 5

A tablet formula may be prepared using the ingredients below:

| Compound | Amount per capsule (mg) | Percent by weight (%) |
|---|---|---|
| Cyclohexyl-3-hydroxy-propyl)-3,4-dimethyl-piperidin-4-yl]-benzamide | 250 | 38 |
| Cellulose microcrystalline | 400 | 60 |
| Silicon dioxide fumed | 10 | 1.5 |
| Stearic acid | 5 | 0.5 |

The components are blended and compressed to form tablets each weighing 665 mg.

### FORMULATION 6

Suspensions each containing 5 mg of medicament per 5 ml dose are made as follows:

| Compound | Amount per 5mL suspension (ml) |
|---|---|
| Cyclohexyl-3-hydroxy-propyl)-3,4-dimethyl-piperidin-4-yl]-benzamide | 5 |
| Sodium carboxymethyl cellulose | 50 |
| Syrup | 1.25 |
| Benzoic acid solution | 0.10 |
| Flavor | q.v. |
| Color | q.v. |
| Water | q.s. to 5mL |

The medicament is passed through a No. 45 mesh U.S. sieve and mixed with the sodium carboxymethylcellulose and syrup to form a smooth paste. The benzoic acid solution, flavor and color is diluted with some of the water and added to the paste with stirring. Sufficient water is then added to produce the required volume.

### FORMULATION 7

An aerosol solution is prepared containing the following components:

| Compound | Concentration by weight (percent) |
|---|---|
| Cyclohexyl-3-hydroxy-propyl)-3,4-dimethyl-piperidin-4-yl]-benzamide hydrochloride | 0.25 |
| Ethanol | 29.75 |
| Propellant 22 (chlorodifluoromethane) | 70.0 |

The active compound is mixed with ethanol and the mixture added to a portion of the Propellant 22, cooled to -30 °C and transferred to a filling device. The required amount is then fed to a stainless steel container and diluted further with the remaining amount of propellant. The valve units are then fitted to the container.

## Claims

1. A compound of the formula I: 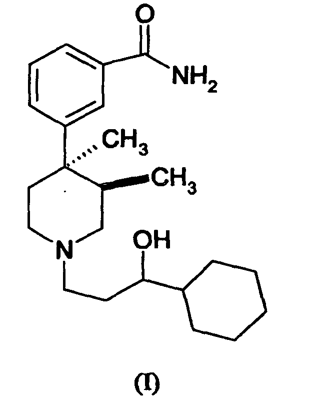 or a pharmaceutically acceptable salt, solvate, enantiomer, racemate, diastereomers or mixtures thereof.

2. The compound of claim 1 which is the (+)-trans isomer.

3. The compound of claim 1 which is trans(+)-1-[-3S-(3-hydroxy-3-cyclohexylpropyl)]-3(R),4(R)-dimethyl-4-(3-phenylcarboxamido)piperidine.

4. A pharmaceutical composition having as an active ingredient an effective amount of a compound of formula I or a pharmaceutically acceptable salt, solvate, enantiomer, racemate, diastereomers or mixtures thereof according to any one of claims 1 to 3.

5. A pharmaceutical composition containing the compound of formula I or a pharmaceutically acceptable salt, solvate, enantiomer, racemate, diastereomers or mixtures thereof according to any one of claims 1 to 3 in association with a carrier and/or diluent

6. Use of a compound of formula I or a pharmaceutically acceptable salt, solvate, enantiomer, racemate, diastereomers or mixtures thereof according to any one of claims 1 to 3, in the manufacture of a medicament for treating or preventing obesity and Related Diseases.

7. Use according to claim 6 wherein the Related Disease is selected from the group consisting of diabetes, diabetic complications, diabetic retinopathy, atherosclerosis, hyperlipidemia, hypertriglycemia, hyperglycemia, and hyperlipoproteinamia.

8. Use of a compound of formula I or a pharmaceutically acceptable salt, solvate, enantiomer, racemate, diastereomers or mixtures thereof according to any one of claims 1 to 3, in the manufacture of a medicament for the treatment of diseases related to obesity including irritable bowel syndrome, nausea, vomiting, depression, smoking and alcohol addiction, sexual dysfunction, substance abuse, drug overdose, addictive behavior disorders, compulsive behaviors, and stroke.

9. Use of a compound of formula I or a pharmaceutically acceptable salt, solvate, enantiomer, racemate, diastereomers or mixtures thereof according to any one of claims 1 to 3, in the manufacture of a medicament for the treatment and/or amelioration of the symptoms associated with obesity and Related Diseases.

10. Use of a compound of formula I or a pharmaceutically acceptable salt, solvate, enantiomer, racemate, diastereomers or mixtures thereof according to any one of claims 1 to 3, in the manufacture of a medicament for the treatment or prevention of obesity and Related Diseases while minimizing undesirable drug-drug interactions in a patient who is also under medication with other drug(s).

11. Use of a compound of formula I or a pharmaceutically acceptable salt, solvate, enantiomer, racemate, diastereomers or mixtures thereof according to any one of claims 1 to 3, in the manufacture of a medicament for appetite suppression.

12. Use of a compound of formula I or a pharmaceutically acceptable salt, solvate, enantiomer, racemate, diastereomers or mixtures thereof according to any one of claims 1 to 3, as an appetite suppressant in a non-therapeutic method of treatment of the human or animal body.

## Patentansprüche

1. Verbindung der Formel I 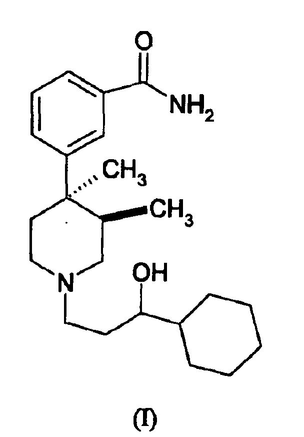 oder ein pharmazeutisch annehmbares Salz, Solvat, Enantiomer, Razemat, Diastereomer oder Gemisch hiervon.

2. Verbindung nach Anspruch 1, die das (+)-trans-Isomer ist.

3. Verbindung nach Anspruch 1, die trans-(+)-1-[3-S-(3-Hydroxy-3-cyclohexylpropyl)]-3(R),4(R)-dimethyl-4-(3-phenylcarboxamido)piperidin ist.

4. Pharmazeutische Zusammensetzung die als Wirkstoff eine wirksame Menge einer Verbindung der Formel I oder eines pharmazeutisch annehmbaren Salzes, Solvats, Enantiomers, Razemats, Diastereomers oder Gemisches hiervon nach einem der Ansprüche 1 bis 3 aufweist.

5. Pharmazeutische Zusammensetzung, die die Verbindung der Formel I oder ein pharmazeutisch annehmbares Salz, Solvat, Enantiomer, Razemat, Diastereomer oder Gemisch hiervon nach einem der Ansprüche 1 bis 3 zusammen mit einem Träger und/oder Verdünnungsmittel enthält.

6. Verwendung einer Verbindung der Formel I oder eines pharmazeutisch annehmbaren Salzes, Solvats, Enantiomers, Razemats, Diastereomers oder Gemisches hiervon nach einem der Ansprüche 1 bis 3 zur Herstellung eines Arzneimittels zur Prävention der Obesität und verwandter Erkrankungen.

7. Verwendung nach Anspruch 6, worin die verwandte Erkrankung aus der Gruppe ausgewählt ist, die besteht aus Diabetes, diabetischen Komplikationen, diabetischer Retinopathie, Atherosklerose, Hyperlipidämie, Hypertriglyceridämie, Hyperglykämie und Hyperlipoproteinämie.

8. Verwendung einer Verbindung der Formel I oder eines pharmazeutisch annehmbaren Salzes, Solvats, Enantiomers, Razemats, Diastereomers oder Gemisches hiervon nach einem der Ansprüche 1 bis 3 zur Herstellung eines Arzneimittels zur Behandlung von Erkrankungen, die mit der Obesität assoziiert sind, einschließlich irritables Darmsyndrom, Übelkeit, Erbrechen, Depression, Rauchen und Alkoholabhängigkeit, Sexualstörung, Substanzmissbrauch, Arzneimittelüberdosis, Abhängigkeitsverhaltensstörungen, kompulsive Verhaltensweisen und Schlaganfall.

9. Verwendung einer Verbindung der Formel I oder eines pharmazeutisch annehmbaren Salzes, Solvats, Enantiomers, Razemats, Diastereomers oder Gemisches hiervon nach einem der Ansprüche 1 bis 3 zur Herstellung eines Arzneimittels zur Behandlung und/oder Linderung der mit Obesität und verwandten Erkrankungen assoziierten Symptomen.

10. Verwendung einer Verbindung der Formel I oder eines pharmazeutisch annehmbaren Salzes, Solvats, Enantiomers, Razemats, Diastereomers oder Gemisches hiervon nach einem der Ansprüche 1 bis 3 zur Herstellung eines Arzneimittels zur Behandlung oder Prävention von Obesität und verwandten Erkrankungen, wobei unerwünschte Arzneimittel-Arzneimittel Wechselwirkungen bei einem Patienten minimiert werden, der auch unter einer Medikation mit anderen Arzneimitteln steht.

11. Verwendung einer Verbindung der Formel I oder eines pharmazeutisch annehmbaren Salzes, Solvats, Enantiomers, Razemats, Diastereomers oder Gemisches hiervon nach einem der Ansprüche 1 bis 3 zur Herstellung eines Arzneimittels zur Appetitunterdrückung.

12. Verwendung einer Verbindung der Formel I oder eines pharmazeutisch annehmbaren Salzes, Solvats, Enantiomers, Razemats, Diastereomers oder Gemisches hiervon nach einem der Ansprüche 1 bis 3 als Appetitzügler in einem nicht-therapeutischen Verfahren des menschlichen oder tierischen Körpers.

## Revendications

1. Composé de formule I : 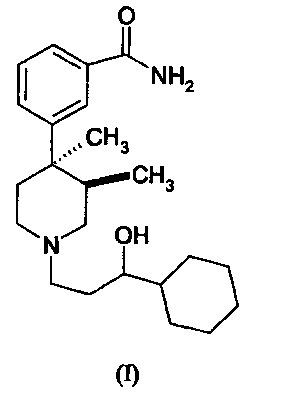 ou sel, solvate, énantiomère, racémate, diastéréoisomères, ou mélanges, de celui-ci, acceptables sur le plan pharmaceutique.

2. Composé selon la revendication 1 qui est l'isomère (+)-trans.

3. Composé selon la revendication 1 qui est la trans(+)-1-[-3S-(3-hydroxy-3-cyclohexylpropyl)]-3(R),4(R)-diméthyl-4-(3-phénylcarboxamido)pipéridine.

4. Composition pharmaceutique ayant en tant qu'ingrédient actif une quantité efficace d'un composé de formule I ou sel, solvate, énantiomère, racémate, diastéréoisomères, ou mélanges, de celui-ci, acceptables sur le plan pharmaceutique selon l'une quelconque des revendications 1 à 3.

5. Composition pharmaceutique contenant le composé de formule 1 ou sel, solvate, énantiomère, racémate, diastéréoisomères, ou mélanges, de celui-ci, acceptables sur le plan pharmaceutique selon l'une quelconque des revendications 1 à 3, en association à un véhicule et/ou un diluant.

6. Utilisation d'un composé de formule I ou d'un sel, solvate, énantiomère, racémate, diastéréoisomères, ou mélanges, de celui-ci, acceptables sur le plan pharmaceutique selon l'une quelconque des revendications 1 à 3 dans la fabrication d'un médicament destiné au traitement ou à la prévention de l'obésité et des maladies apparentées.

7. Utilisation selon la revendication 6 dans laquelle la maladie apparentée est choisie dans le groupe formé par le diabète, les complications du diabète, la rétinopathie diabétique, l'athérosclérose, l'hyperlipidémie, l'hypertriglycémie, l'hyperglycémie, et l'hyperlipoprotéinémie.

8. Utilisation d'un composé de formule I ou d'un sel, solvate, énantiomère, racémate, diastéréoisomères, ou mélanges, de celui-ci, acceptables sur le plan pharmaceutique selon l'une quelconque des revendications 1 à 3 dans la fabrication d'un médicament destiné au traitement de maladies liées à l'obésité, incluant le syndrome du côlon irritable, la nausée, le vomissement, la dépression, le tabagisme et l'alcoolisme, le dysfonctionnement sexuel, la toxicomanie, l'overdose de drogue, les troubles du comportement de la pharmacodépendance, les comportements compulsifs, et l'attaque.

9. Utilisation d'un composé de formule I ou d'un sel, solvate, énantiomère, racémate, diastéréoisomères, ou mélanges, de celui-ci, acceptables sur le plan pharmaceutique selon l'une quelconque des revendications 1 à 3, dans la fabrication d'un médicament destiné au traitement et/ou à l'amélioration des symptômes associés à l'obésité et aux maladies apparentées.

10. Utilisation d'un composé de formule I ou d'un sel, solvate, énantiomère, racémate, diastéréoisomères, ou mélanges, de celui-ci, acceptables sur le plan pharmaceutique selon l'une quelconque des revendications 1 à 3 dans la fabrication d'un médicament destiné au traitement ou à la prévention de l'obésité et des maladies apparentées, tout en minimisant les interactions médicament-médicament indésirables, chez un patient se trouvant sous médication avec un autre ou d'autres médicaments.

11. Utilisation d'un composé de formule I ou d'un sel, solvate, énantiomère, racémate, diastéréoisomères, ou mélanges, de celui-ci, acceptables sur le plan pharmaceutique selon l'une quelconque des revendications 1 à 3, dans la fabrication d'un médicament destiné à supprimer l'appétit.

12. Utilisation d'un composé de formule I ou d'un sel, solvate, énantiomère, racémate, diastéréoisomères, ou mélanges, de celui-ci, acceptables sur le plan pharmaceutique selon l'une quelconque des revendications 1 à 3, en tant qu'agent supprimant l'appétit dans un mode de traitement non thérapeutique du corps humain ou animal.
